# EUROPÄISCHE PATENTSCHRIFT

(11) **EP 1 996 128 B1**
(45) Veröffentlichungstag und Bekanntmachung des Hinweises auf die Patenterteilung: **21.10.2009**
(21) Anmeldenummer: 07711243.1
(22) Anmeldetag: 15.03.2007
(51) Int. Cl.: A61F 2/50, A61F 2/64, A61F 2/68, A61F 2/72

(54) **GELENKEINRICHTUNG**
JOINT DEVICE
DISPOSITIF D'ARTICULATION

(30) Priorität: 17.03.2006 DE 102006012716
(43) Veröffentlichungstag der Anmeldung: 03.12.2008
(73) Patentinhaber: Otto Bock HealthCare IP GmbH & Co. KG, 37115 Duderstadt (DE)
(72) Erfinder: AUBERGER, Roland, A-1020 Wein (AT)
(74) Vertreter: Stornebel, Kai
(86) Internationale Anmeldenummer: PCT/DE2007/000499
(87) Internationale Veröffentlichungsnummer: WO 2007/107150

(56) Entgegenhaltungen:
- EP-A- 1 159 936
- DE-C- 295 807
- DE-C- 329 908
- DE-U1- 9 320 708
- GB-A- 2 244 006

## Beschreibung

Die Erfindung betrifft eine Gelenkeinrichtung für Orthesen oder Prothesen mit einem Oberteil und einem um eine Schwenkachse drehbar daran gelagerten Unterteil und einer Bremseinrichtung, die eine Schwenkbewegung des Unterteils relativ zu dem Oberteil abbremst oder blockiert. Eine Gelenkeinrichtung gemäß den Merkmalen des Oberbegriffs des Anspruchs 1 ist aus der DE-C-329908 bekannt.

Aus dem Stand der Technik sind eine Vielzahl von Gelenkeinrichtungen für Orthesen und Prothesen bekannt, die eine verschwenkbare Lagerung zweier Prothesen- oder Orthesenkomponenten gegeneinander ermöglichen. Bei einer Anordnung der Gelenkeinrichtung beispielsweise als Kniegelenk ist es wünschenswert, eine Dämpfung in der Schwungphase und in der Standphase einstellen zu können.

Eine Anordnung einer Gelenkeinrichtung in einem einachsigen Schultergelenk sollte eine Verriegelung einer Armprothese oder Orthese gegen ein Herunterfallen ermöglichen. Gleiches gilt für die Anordnung der Gelenkeinrichtung in einem Ellenbogengelenk.

Zur Einrichtung der Bremseinrichtung in diesen Gelenken sind verschiedene Wirkprinzipien möglich, neben einer formschlüssigen Verriegelung in vorbestimmten Rastpositionen besteht die Möglichkeit einer hydraulischen Dämpfung bzw. Abbremsung der Bewegungen. Solche hydraulischen Steuerungen sind jedoch technologisch sehr aufwendig und sind hinsichtlich Raumbedarf und Gewicht nachteilig.

Aufgabe der vorliegenden Erfindung ist es, eine Gelenkeinrichtung zu entwickeln, die mit einer geringen Schaltkraft geöffnet oder gesperrt werden kann und gleichzeitig einen sehr geringen Bauraum benötigt. Insbesondere soll die medial-laterale Ausdehnung so gering wie möglich gehalten werden, um bei der Anwendung in einer Orthese ein unauffälliges Tragen unter der Kleidung zu ermöglichen.

Erfindungsgemäß wird diese Aufgabe durch eine Gelenkeinrichtung mit den Merkmalen des Anspruchs 1 gelöst. Vorteilhafte Ausgestaltungen und Weiterbildungen der Erfindung sind in den Unteransprüchen aufgeführt.

Die erfindungsgemäße Gelenkeinrichtung für Orthesen oder Prothesen mit einem Oberteil und einem um eine Schwenkachse drehbar daran gelagerten Unterteil und einer Bremseinrichtung, die eine Schwenkbewegung des Unterteils relativ zu dem Oberteil abbremst oder blockiert, sieht vor, dass eine Spiralfeder zwischen zwei Anlaufflächen angeordnet ist und um eine Verdrehachse parallel zu der Schwenkachse in Richtung auf die Anlaufflächen verspannbar gelagert ist, wobei bei einer Verspannung in Richtung auf die Anlaufflächen die Spiralfeder in Kontakt mit diesen tritt. Dadurch werden die Seitenflanken der Spiralfeder an die Anlaufflächen angepresst. Dadurch wird durch Reibung der Aufbau eines sehr hohen Bremsmomentes bei sehr geringen Schaltkräften und Schaltwegen ermöglicht. Aufgrund der Ausbildung als eine Spiralfeder ist es möglich, eine sehr geringe Baugröße in medial-lateraler Ausdehnung zu ermöglichen, auch wenn ein relativ großer Durchmesser dafür in Kauf genommen werden muss. Durch das Verspannen der Spiralfeder in Richtung auf die Anlaufflächen versuchen die einzelnen Spiralgänge in Richtung auf die Anlaufflächen zu wandern. Aufgrund der Anlaufflächen ist dies jedoch nicht möglich, so dass sich die Spirale verkeilt und ein Brems- bzw. Sperrmoment realisiert werden kann. Durch den Keileffekt wird die Verspannung verstärkt, so dass ein sich selbst verstärkendes System vorliegt. Wird eine Relativbewegung des Ober- und Unterteils entgegen der Windungsrichtung ausgeführt, tendieren die Spiralgänge von den Anlaufflächen weg, so dass eine beinahe ungehinderte Bewegung stets möglich ist, sofern die Reibung aufgrund der Vorspannung nicht so groß ist, dass die Öffnungsbewegung zumindest kompensiert wird.

Eine Weiterbildung der Erfindung sieht vor, dass die Anlaufflächen konturiert ausgebildet sind, insbesondere radial konisch oder radial gekrümmt, um die Reibfläche zu erhöhen und um ihre radial nach außen hintereinander liegenden Spiralgänge der Spiralfeder nutzbar zu machen. Dadurch wird ein progressives Bremsverhalten erreicht. Die Spiralfeder ist dabei bevorzugt entsprechend zu den Anlaufflächen konturiert, um eine möglichst große Reibfläche zwischen den Anlaufflächen und den Spiralfederflanken bereitzustellen. Eine konische bzw. konstant gekrümmte Ausgestaltung der Anlaufflächen und der Spiralfederkontur ermöglicht einen zusätzlichen Keileffekt, der eine hohe Kraftverstärkung bewirkt. Ebenfalls vorgesehen sind unstetige Querschnittgeometrien, z.B. mehrfach konische oder gekrümmte Konturen, ähnlich der eines Tannenbaumes. Dadurch lässt sich die Baubreite bei einem gegebenen Keilwinkel reduzieren.

Eine besonders kompakte Bauweise wird ermöglicht, wenn die Verdrehachse der Spiralfeder mit der Schwenkachse des Gelenks zusammenfällt.

Bei einer konischen Ausgestaltung der Anlaufflächen ist ein Winkel zwischen 10° und 50° bevorzugt. Kleinere Winkel bergen die Gefahr einer Selbsthemmung, größere Winkel vergrößern den Bauraum in medial-lateraler Richtung und reduzieren die Selbstverstärkung, was nicht gewünscht ist.

Die Spiralfeder ist in ihrer Spannung bevorzugt einstellbar und kann in ihrer Ruhestellung in Kontakt mit den Anlaufflächen liegen. Dadurch wird sichergestellt, dass in derjenigen Schwenkrichtung, die gesperrt werden soll, aufgrund des sich selbst initiierenden und verstärkenden Bremseffektes stets eine sichere Bremswirkung gewährleistet ist, auch wenn zum Bewegen der Gelenkeinrichtung, entgegen dieser Richtung, ein gewisses Moment aufgebracht werden muss, um die initiale Bremswirkung der Spiralfeder zu überwinden.

Eine Weiterbildung der Erfindung sieht vor, dass die Spiralfeder mit einem Aktuator gekoppelt ist, der die Spiralfeder spannt oder entspannt. Dadurch ist es möglich, eine aktive Steuerung der Gelenkeinrichtung vorzunehmen, beispielsweise indem durch eine gewollte Entspannung der Spiralfeder, also ein Aufweiten oder Zusammenziehen der Spiralfeder von den Anlaufflächen weg, eine stets freie Beweglichkeit gegeben ist. Andererseits kann durch eine hinreichend große Verspannung in Richtung auf die Anlaufflächen eine ständige und sichere Verriegelung der Gelenkeinrichtung bzw. Abbremsung der Verschwenkbewegung erfolgen.

Der Aktuator ist bevorzugt ein Stell- oder Servomotor, der zur Verstärkung der Stellkraft und der Verspannung der Spiralfeder in Richtung auf die Anlaufflächen oder von diesen weg mit einem Hebel gekoppelt sein kann, der wiederum auf die Spiralfeder einwirkt. In einer Ausgangsstellung kann der Hebel federbelastet in Verriegelungsrichtung sein, um eine Verschleißkompensation durchzuführen. Dies ist insbesondere durch eine Spannfeder leicht zu realisieren. Um bei einem Ausfall des Aktuators eine sichere Abbremsung oder Verriegelung zu gewährleisten, also um eine ausfallsichere Gelenkeinrichtung bereitzustellen, ist die Spiralfeder gegen den Aktuator in Verriegelungsrichtung vorgespannt. Fällt der Aktuator aus, verdreht sich die Spiralfeder selbstständig in Richtung auf die Anlaufflächen, was eine initiale Bremswirkung zur Folge hat, die durch die Selbstverstärkung und somit die Verspannung der Spiralfeder gegen die Anlaufflächen verstärkt wird. Dadurch ist es möglich, eine Schwenkrichtung bei Ausfall des Aktuators stets zu sperren. Bei einem Kniegelenk bzw. einer Kniegelenksorthese würde eine Sperrung der Flexion erfolgen, um ein unbeabsichtigtes Einknicken zu vermeiden.

Eine Weiterbildung der Erfindung sieht vor, dass eine Sensoreinrichtung vorhanden ist, die Momente, Winkelstellungen der Ober- und Unterteile zueinander oder auf die Gelenkeinrichtung wirkenden Kräfte misst. Diese Sensoreinrichtung ist mit einer Steuereinheit gekoppelt, die in Abhängigkeit von den gemessenen Größen bzw. Parametern den Aktuator aktiviert und die Spiralfeder gegenüber den Anlaufflächen verspannt oder entspannt. Dadurch wird belastungsabhängig und stellungsabhängig eine Sperrung oder ein Lösen der Bremseinrichtung bewirkt, wodurch eine situationsangepasste Bremsung der Gelenkeinrichtung erfolgt. Beispielsweise kann bei einem Stolpern eines Prothesenträgers oder Orthesenträgers die Bremseinrichtung aktiviert und die Gelenkeinrichtung verriegelt werden, so dass ein unbeabsichtigtes Einknicken verhindert wird. Die Kniebeugung kann somit gezielt limitiert werden.

Eine Weiterbildung der Erfindung sieht vor, dass der Aktuator mit einer Steuereinheit gekoppelt ist, die über myoelektrische Signale eine Aktivierung oder Deaktivierung des Aktuators bewirkt. So können Gelenke in einer bestimmten Stellung aufgrund myoelektrischer Signale verriegelt oder entriegelt werden.

Ebenfalls ist es möglich, dass eine Einrichtung zur Erfassung der räumlichen Orientierung des Oberteils und/oder des Unterteils mit dem Aktuator gekoppelt ist. Auch kann eine Einrichtung zur Erfassung der auftretenden Beschleunigungen des Oberteils und/oder des Unterteils oder der daran befestigten Einrichtungen mit dem Aktuator gekoppelt sein. Dadurch ist es möglich, dass die Orthese/Prothese oder die Einrichtung, in der die Gelenkeinrichtung eingesetzt wird, mit Hilfe eines Absoultwinkelsignals, also einer Orientierung entweder des Oberteils oder des Unterteils zum Lot, angesteuert werden kann. Durch die Stellung des Oberteils und des Unterteils zueinander ist der Gelenkwinkel, beispielsweise der Kniewinkel bekannt, so dass auch die absolute Orientierung im Raum sowohl vom Oberteils als auch vom Unterteil bzw. der daran befestigten Einrichtungen bekannt ist. Die Einrichtung kann also als ein Absolutwinkelsensor an dem Oberteil oder dem Unterteil oder an einer daran befestigten Einrichtung ausgestattet sein. Dadurch ist es möglich, die Ansteuerung des Aktuators aufgrund von Informationen zur räumlichen Orientierung der Gelenkeinrichtung bzw. des Systems, in dem die Gelenkeinrichtung angeordnet ist, durchzuführen. Ebenso ist es möglich, diese Ansteuerung auf der Grundlage der auftretenden und gemessenen Beschleunigungen vorzunehmen. Somit kann in Abhängigkeit von dem Absolutwinkel und der auftretenden Beschleunigungen die Bremse aktiviert oder außer Eingriff geschaltet werden.

Bei einer mehrgängigen Ausgestaltung der Spiralfeder ist eine besonders leichte Verstellbarkeit gewährleistet. Ein damit einhergehender Verlust an Reibfläche kann durch eine Durchmesservergrößerung der Spiralfeder und Anlaufflächen kompensiert werden.

Über eine nichtlineare Spiralgeometrie ist es möglich, ein progressives Bremsverhalten zu erreichen. Ebenfalls ist es möglich, über die Anzahl der Spiralwindungen das Betätigungsmoment zu variieren. Je mehr Windungen eine Spiralfeder aufweist, desto länger ist der Betätigungsweg, allerdings lässt sich dadurch das Bremsmoment sehr gut einstellen. Um eine gewisse Einstellungstoleranz und Aktivierungsweichheit realisieren zu können, kann eine Anlauffläche begrenzt axial verschieblich gelagert sein. Die Spiralgeometrie ist nicht auf stetig gekrümmte Freiräume bzw. Spiralgänge beschränkt. Es können auch Spiralgänge mit sich verändernden Querschnitten, Steigungen, Orientierungen oder dergleichen vorgesehen sein. Auch blockartige Elemente können vorgesehen werden.

Nachfolgend wird ein Ausführungsbeispiel der Erfindung anhand der Figuren näher erläutert. Es zeigen:
- Figur 1 -: eine Gelenkeinrichtung in einem zusammengebauten Zustand;
- Figur 2 -: eine Unteransicht einer Gelenkeinrichtung gemäß Figur 1;
- Figur 3 -: eine Draufsicht auf eine teilgeschnittene Gelenkeinrichtung;
- Figur 4 -: eine Schnittdarstellung durch eine fertig montierte Gelenkeinrichtung;
- Figur 5 -: eine vergrößerte Detaildarstellung des Ausschnittes A der Figur 4
- Figur 6 -: verschiedene Ansichten einer mehrgängigen Euler'schen Spiralfeder;
- Figur 7 -: verschiedene Ansichten einer nichtlinearen Spiralfeder; sowie
- Figur 8 -: eine Variante der Gelenkeinrichtung in Teildarstellung.

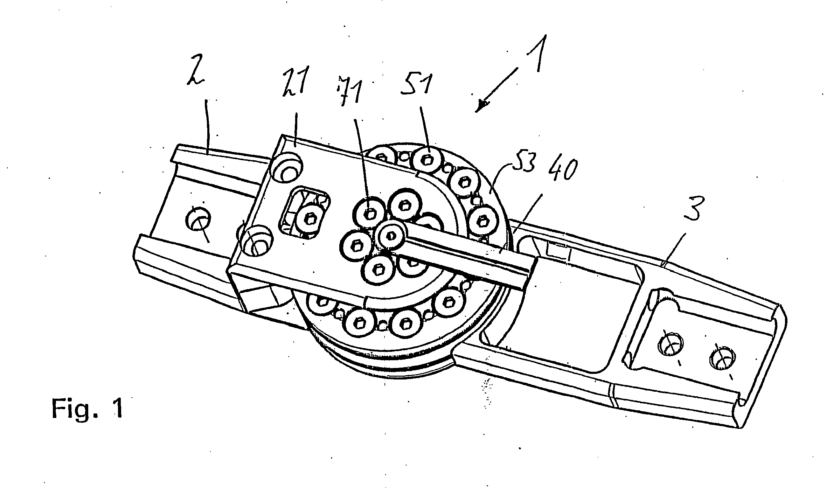

Figur 1 zeigt in einer Draufsicht eine fertig montierte Gelenkeinrichtung 1 mit einem Oberteil 2 und einem Unterteil 3. Die Bezeichnung Oberteil und Unterteil bedeutet keine technische Limitierung auf eine konkrete Anordnung, vielmehr kann ein Oberteil sowohl am proximalen als auch distalen Ende einer Prothese oder Orthese angeordnet sein, gleiches gilt entsprechend für das Unterteil.

Das Oberteil 2 weist einen L-förmigen Bügel 21 auf, der auf dem Oberteil 2 axial verschiebbar verbunden, angeschraubt oder aufgesetzt ist. Über Schrauben 71 ist eine Anlaufflächen, die in dieser Figur nicht dargestellt ist, an dem Bügel 21 fest angeschraubt. Vor dem Bügel 21 ragt ein Betätigungshebel 40 in Richtung auf das Unterteil 3 und kann durch einen nicht dargestellten Aktuator in beide Richtungen um eine Schwenkachse 4 verschwenkt werden. Der Hebel 40 ist relativ zu dem Oberteil 2 verschwenkbar gelagert und bewegt eine Spiralfeder, die drehfest an dem Unterteil 3 über Schrauben 51 an dem Unterteil 3 befestigt ist. Das Oberteil 2 und das Unterteil 3 können an Befestigungselementen von Orthesen oder Prothesen befestigt werden.

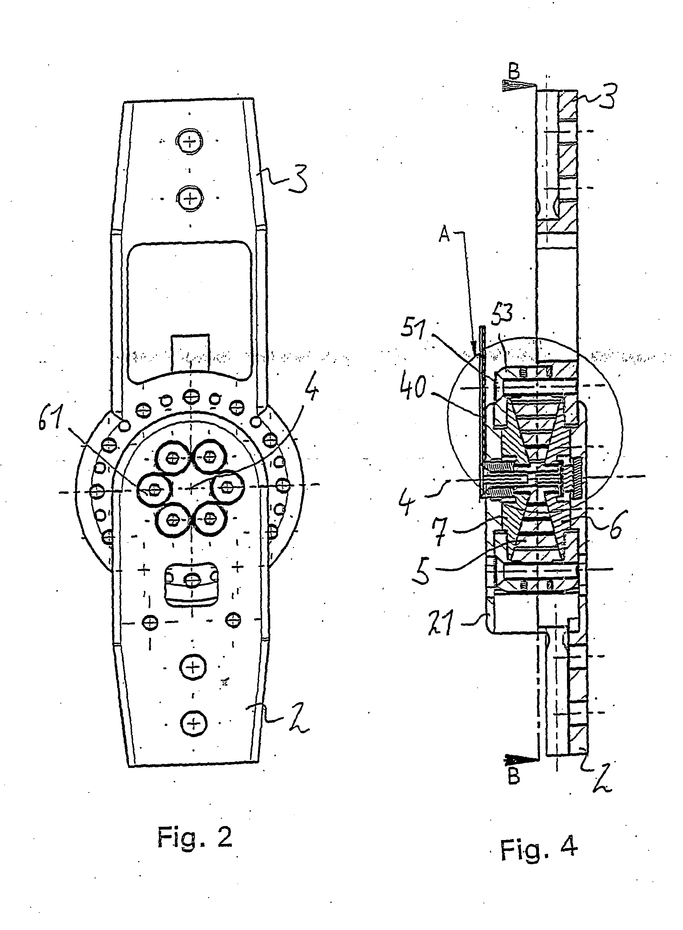

In der Figur 2 ist eine Unteransicht der Gelenkeinrichtung 1 dargestellt. Das Oberteil 2 bildet eine Trägerfläche aus, an der über Schrauben 61 die zweite Anlauffläche drehfest gelagert und zu einer funktionellen Einheit verbunden ist. Das Unterteil 3 ist relativ zu dem Oberteil 2 um eine zentrale Schwenkachse 4 verschwenkbar. An dem Unterteil 3 ist ein kreisförmiges Kopfstück ausgebildet, an dem die Spiralfeder über die Schrauben 51 festgelegt wird.

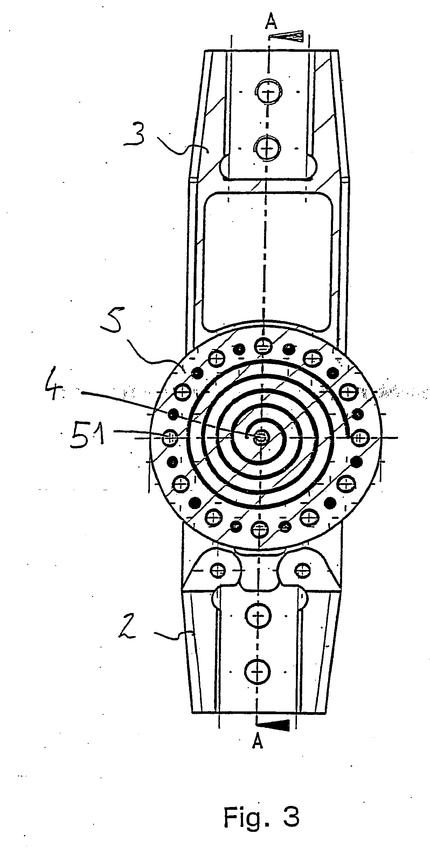

Eine mögliche Ausgestaltung der Spiralfeder 5 ist in der Figur 3 gezeigt, die eine Schnittdarstellung durch das Oberteil 2, das Unterteil 3 und die Spiralfeder 5 zeigt. Der Bügel 21, der Hebel 40 und der obere kreisförmige Deckel 53 sind nicht gezeigt.

Die Spiralfeder 5 ist über die am Umfang verteilten Schrauben 51 an dem Unterteil 3 drehfest gelagert. Sie hat einen kreisförmigen Querschnitt mit einem eingängigen Spiralgang, der sich von außen bis zur Drehachse 4, entgegen dem Uhrzeigersinn, spiralförmig nach innen erstreckt. Parallel zu der Schwenkachse 4 und mit dieser zusammenfallend kann die Spiralfeder 5 durch eine Betätigung des Hebels 40 entgegen dem Uhrzeigersinn in Windungsrichtung verspannt werden, so dass sich die Spiralfeder 5 nach innen in Richtung auf die Drehachse 4 zusammenzieht. Die einzelnen Spiralgänge wandern nach innen und legen sich dann an die Anlaufflächen 6, 7, die drehfest über die Schrauben 61, 71 an dem Oberteil 2 gelagert sind. Je nach Ausgestaltung der Anlaufflächen 6, 7 sowie der Spiralfeder 5 ist es möglich, bei einer Drehung in oder entgegen der Windungsrichtung ein Bremsen zu erreichen.

Anhand der Figur 4 kann der konstruktive Aufbau und die Wirkungsweise der Gelenkeinrichtung 1 besonders gut erkannt werden. In der Schnittdarstellung gemäß Figur 4 sind das Oberteil 2 und das Unterteil 3 gegeneinander schwenkbar um die Schwenkachse 4 verlagerbar aneinander befestigt. An dem Oberteil 2 mit dem über Schrauben oder Bolzen befestigten Bügel 21 sind Anlaufflächen 6, 7 über die Schrauben 61, 71 drehfest gelagert. Die Anlaufflächen 6, 7 weisen eine konische Kontur auf und sind so zueinander angeordnet, dass sich radial nach außen ein sich erweiternder Keilraum ausbildet. Innerbalb dieses Keilraumes ist die Spiralfeder 5 angeordnet, die drehfest durch die an ihrem Umfang verteilten Schrauben 51 an dem Unterteil 3 befestigt ist. Die Spiralfeder 5 liegt einerseits unmittelbar auf dem Unterteil 3 und andererseits auf dem Deckel 53 auf. Der Hebel 40 ist ebenfalls schwenkbar um die Drehachse 4, die gleichzeitig die Verspannachse ist, gelagert und wirkt auf das Zentrum der Spiralfeder 5. Wird der Hebel 40, wie in der Figur 1 gezeigt, entgegen dem Uhrzeigersinn betätigt, verspannt sich die Spiralfeder 5 nach innen. Die einzelnen Spiralgänge mit ihrer konischen Kontur versuchen, nach innen zu wandern. Dies ist aber wegen der lateralen Begrenzungen durch die Anlaufflächen 6, 7, die als Bremsscheiben wirken, nicht möglich. Die seitlichen Flächen der Spiralfeder 5 verkeilen sich gegen die Anlaufflächen 6, 7, wodurch ein Brems- bzw. ein Sperrmoment realisiert wird. Wird hingegen der Hebel in Uhrzeigersinn entgegen der Windungsrichtung bewegt, weiten sich die Spiralgänge nach außen auf. Dadurch wird die Reibung zwischen den seitlichen Spiralflächen und den Anlaufflächen 6,7 verringert oder aufgehoben, so dass im Extremfall kein Kontakt mehr zwischen der Spiralfeder 5 und den Anlaufflächen 6,7 besteht. Das Gelenk bzw. die Gelenkenrichtung 1 lässt sich somit frei bewegen. Wird ein eigentlich gesperrtes Gelenk bzw. eine gesperrte Gelenkeinrichtung 1 entgegen der Windungsrichtung der Spiralfeder 5 bewegt, so bewirkt die Reibung zwischen den Seitenflächen der Spiralfeder 5 und den Anlaufflächen 6, 7 ein Aufweiten der Spiralgänge, so dass sich das Gelenk in dieser Richtung stets frei bewegen lässt, während es in einer entgegengesetzten Bewegungsrichtung automatisch sperrt.

Bei dem dargestellten Prinzip wirkt die zum Aufbau des Brems- oder Sperrmomentes notwendige Normakraft senkrecht zu der jeweiligen Keilfläche. Um die Belastungen auf die Bauteile, wie Spiralfeder 5 und Anlaufflächen 6, 7 bei einem vorgegebenen Bremsmoment zu reduzieren, ist es empfehlenswert, möglichst viele Reibflächen zu realisieren. Vorliegend sind zwei Anlaufflächen 6, 7 vorgesehen. Diese Anlaufflächen 6, 7 werden beim Sperren der Gelenkeinrichtung 1 auseinandergedrückt und reiben somit an der Feder 5.

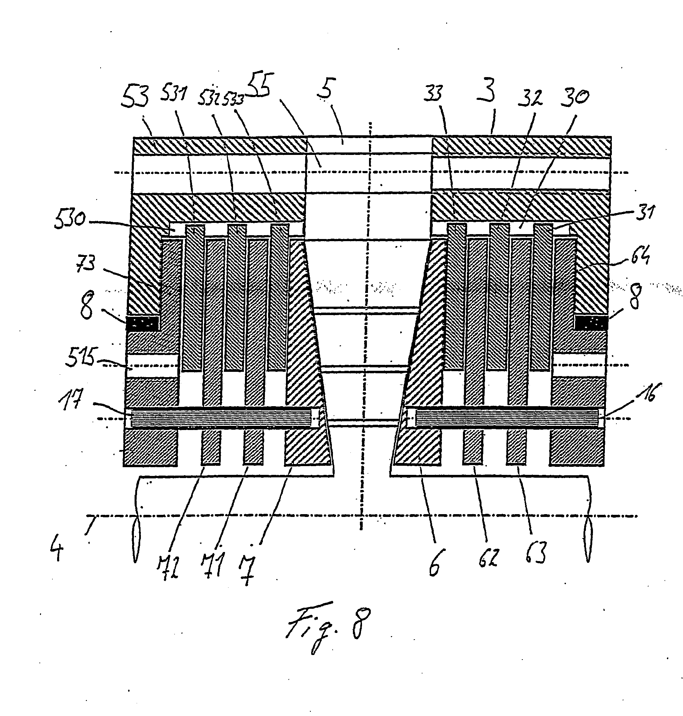

Sind die beiden Anlaufflächen 6, 7 begrenzt axial verschiebbar gelagert, so bewirkt die radial nach innen gerichtete Bewegung der Spiralgänge zusätzlich noch Reibung der außen liegenden Reibflächen 36, 537 der Anlaufflächen 6, 7 an dem Unterteil 3 und dem kreisförmigen Deckel 53. Dadurch entstehen insgesamt vier Bremsflächen, was bei einem vorgegebenen, zu realisierenden Bremsmoment eine Entlastung für die Feder 5 bedeutet. Die Anzahl der Reibflächen lässt sich weiter erhöhen, indem man die Anlaufflächen 6, 7, bzw. das Unterteil 3 und den Deckel 53 mit einem Lamellenstapel, wie er in der Figur 8 gezeigt ist, verbindet. Ebenfalls ist es möglich, mehrere Anlaufflächen und Spiralfedern axial nebeneinander zu lagern. Sieht man zwei Spiralfedern mit unterschiedlicher Windungsrichtung vor, lässt sich das Gelenk in beiden Bewegungsrichtungen gleichzeitig sperren.

Über den Hebel 40 kann, wie bereits ausgeführt, die Spiralfeder 5 verspannt oder entspannt werden, wodurch das Gelenk bzw. die Gelenkeinrichtung 1 entweder geöffnet oder gesperrt wird. Als Hebel 40 kann dabei auch ein Zahnrad oder ein Zahnradsegment dienen. Über ein Zahnradgetriebe kann dann eine unmittelbare Kopplung eines Servomotors als Aktuator mit dem Zahnrad und darüber mit der Spiralfeder oder den Spiralfedern erfolgen, wodurch der erforderliche Bauraum reduziert werden kann. Eine Anpassung an sich verändernde Kraftverhältnisse kann statt über eine Änderung der Hebellänge bei einem Betätigungshebel über eine Veränderung der Übersetzung erfolgen.

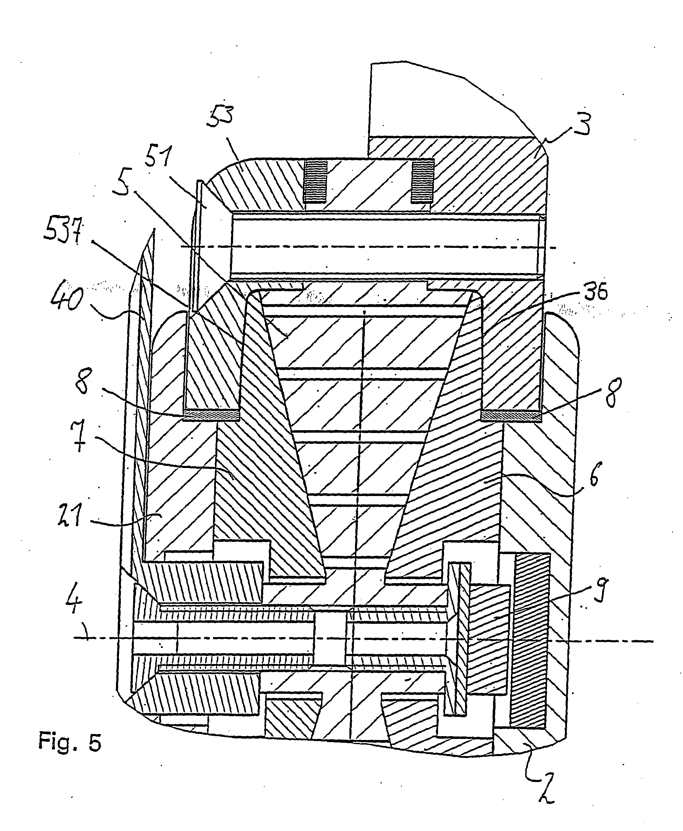

In der Teildarstellung der Figur 5 ist weiterhin ein Radialgleitlager 8 zwischen dem Oberteil 2, 21 und dem Unterteil 3 vorgesehen, das an Stirnflächen der Anlaufflächen 6, 7 und dem Oberteil 2 und dem Bügel 21 anliegt. Das Radiallager 8 muss nicht an den Stirnflächen anliegen, wichtig ist, dass es gegen allzu große axiale Verschiebungen gesichert ist. Dadurch wird bei einer Nichtverspannung der Spiralfeder 5 ein Gleiten um die Schwenkachse 4 ermöglicht. In der Figur 5 ist ebenfalls ein Winkelsensor 9 zu erkennen, der die Winkelstellung des Oberteils 2 zu dem Unterteil 3 sensiert und diese Daten an eine Steuereinrichtung weitergibt, die wiederum einen nicht dargestellten Aktuator, beispielsweise einen Stell- oder Servomotor, betätigt. Dadurch kann die Gelenkeinrichtung 1 gesperrt oder geöffnet werden. In dem Oberteil 2 oder bevorzugt in dem Unterteil 3 kann zudem ein Momentensensor in der Struktur integriert sein.

Statt über die Schrauben 51 kann der Deckel 53 über ein Gewinde unmittelbar mit dem Unterteil 3 verschraubt sein, wobei eine Verdrehsicherung vorgesehen ist. Wie über die Schrauben 51 kann über das Gewinde das Spaltmaß eingestellt und die Teile zusammengehalten werden.

Um eine nicht gewollte Verspannung zu vermeiden, kann es notwendig sein, dass das Oberteil 2 und der daran befestigte Bügel 21 ein axiales Spiel zulassen, so dass die beiden Brems- oder Anlaufflächen 6, 7 axial zueinander leicht verschiebbar sind. Dann ist es vorgesehen, dass der Bügel 21 mit Spiel auf dem Oberteil 2 befestigt oder über zwei Bolzen, die eine axiale Verschiebung zulassen, miteinander verbunden sind.

Die Geometrie der Spiralkurve der Spiralfeder 5 hat einen starken Einfluss auf die Charakteristik der Gelenkreinrichtung 1. Spiralen mit vielen Windungen haben einen längeren Betätigungsweg als Spiralen mit wenigen Windungen, allerdings lässt sich das realisierte Bremsmoment wesentlich feiner einstellen. Nichtlineare Spiralgeometrien sind möglich, um ein z.B. ein progressives Bremsverhalten zu erreichen oder um die Belastung der Spiralfeder zu optimieren.

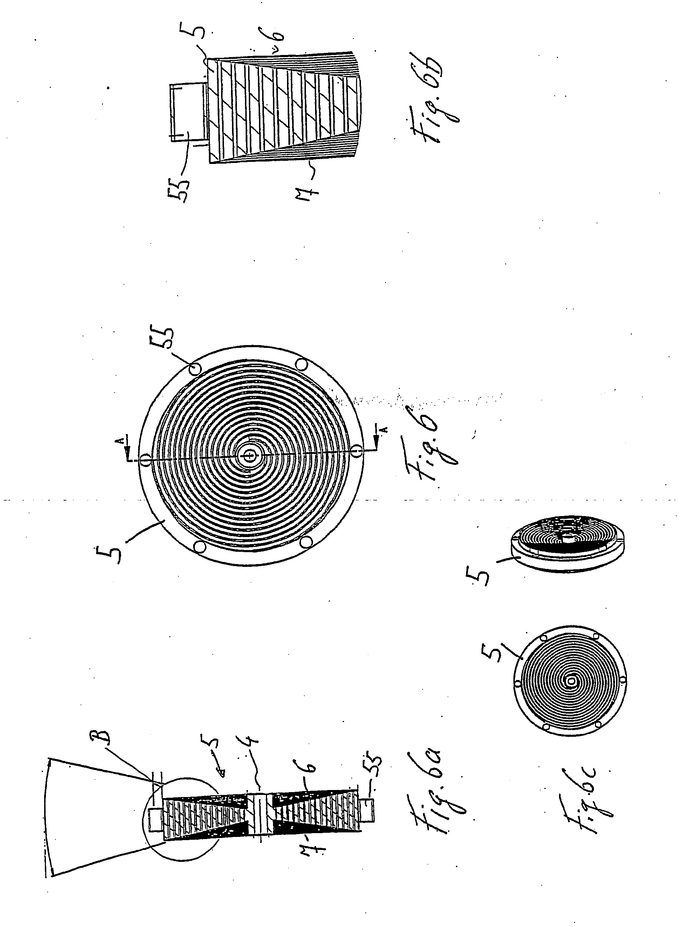

Um das Betätigungsmoment über den Hebel 40 zu reduzieren, können mehrere Windungen parallel zueinander geschnitten werden, also ein mehrgängiger Spiralgang ausgebildet werden, wie er z.B. in der Figur 6 gezeigt ist.

Bei einer Anwendung in Kniegelenksorthesen oder -prothesen ist es vorgesehen, dass die Gelenkeinrichtung 1 in einem Verriegelungsmodus eingestellt ist, der garantiert, dass das Gelenk bei einem Ausfall des Aktuators nicht frei verschwenkbar ist. Dies kann durch eine Vorspannung des Betätigungshebels 40, beispielsweise über eine Feder, in eine gesperrte Position erreicht werden. Das Öffnen der Gelenkeinrichtung bzw. der Bremseinrichtung erfolgt dann aktiv gegen eine Federkraft, beispielsweise auch gegen eine Vorspannung über die Spiralfeder 5. Alternativ zu einer Vorspannung über die Spiralfeder 5 kann eine separate Feder vorgesehen sein, die gleichzeitig eine automatische Verschleißkompensation bewirkt. Das Öffnen der Gelenkeinrichtung 1 erfolgt dann aktiv entgegen der Federkraft. Wenn die Vorspannung entgegen dem Aktuator in der Mitte des interessierenden Momentenbereiehes gewählt ist, kann das zu realisierende Bremsmoment weiter verstärkt bzw. abgeschwächt werden.

Ebenfalls ist es möglich, eine größere Anzahl von Anlaufflächen 6, 7, beispielsweise hintereinander gestapelt, wie bei einer Lamellenkupplung, vorzusehen, um die Belastung der Spiralfeder 5 weiter zu verringern. Ebenfalls ist es möglich, statt einer geradkonischen Ausbildung der Anlaufflächen 6, 7 eine Krümmung vorzusehen, um einen besonderen Anstieg des Bremsmomentes bei einer Verlagerung der Spiralgänge nach innen zu ermöglichen.

Sind die Anlaufflächen 6, 7 so ausgebildet, dass sie einen sich nach radial außen verjüngenden Keilraum ausbilden, sind die Drehrichtungen entsprechend umzukehren, da durch eine Aufweitung der Spiralfeder 5 das Anlegen an die Anlaufflächen 6, 7 bewirkt und das Bremsmoment realisiert werden kann.

Mit der dargestellten Ausführungsform ist ein Öffnen der Gelenkeinrichtung unter Last durch einen motorischen Aktuator oder aber auch durch eine manuelle Betätigung leicht möglich. Im Notfall und bei Ausfall eines Aktuators wird die Gelenkeinrichtung in eine Schwenkrichtung stets gesperrt, wenn in der Ausgangsstellung die Spiralfeder 5 in Kontakt mit den Anlaufflächen 6, 7 steht. Durch eine Verschwenkung der Ober- und Unterteile 2, 3 zueinander erfolgt dann eine Realisierung eines Bremsmomentes und eine Verstärkung der Bremswirkung durch ein Verkeilen.

Alternativ zu der dargestellten Ausbildung mit nur einer Spiralfeder 5 können zwei Spiralfedern axial nebeneinander in entgegengesetzter Richtung wirkend angeordnet sein, so dass eine in beide Schwenkrichtungen wirksame Bremse erhalten werden kann, die wenig Bauteile erfordert und in beiden Bewegungrichtungen unter Last schaltbar ist.

Die oben beschriebene Gelenkeinrichtung kann auch bei beliebig oft durchdrehenden Komponenten eingesetzt werden und ist somit auch für Überholkupplungen oder Rücklaufsperren einsetzbar.

In der Figur 6 ist eine Draufsicht auf eine Spiralfeder 5 mit Euler'schen Spiralgängen, also einer linearen Spiralgeometrie gezeigt. Insgesamt sind vier Spiralgänge mit jeweils dreieinhalb Windungen vorgesehen, so dass sich aufgrund der relativ großen Fläche der Spiralgänge eine relativ leichte Verstell- und Verdrehbarkeit der Spiralfeder 5 ergibt. In der Figur 6a, in der ein Schnitt entlang der Linie A-A in der Figur 6 dargestellt ist, ist die Anordnung der Spiralfeder 5 zwischen zwei Anlaufflächen 6, 7 zu erkennen. An dem äußeren Umfang der Spiralfeder 5, vorliegend symmetrisch einander gegenüberliegend, sind Bohrungen 55 ausgebildet, durch die die Befestigungsschrauben oder Bolzen 51 hindurch geführt werden können, um die Spiralfeder an dem Unterteil 3 festzulegen. In der Figur 6a ist der Keilwinkel α der Spiralfeder 5 eingezeichnet, der zwischen 10° und 50°, vorliegend bei ungefähr 30° liegt. Der Keilwinkel α ist gleichzeitig der Keilwinkel, der zwischen den Anlaufflächen 6, 7 ausgebildet ist.

Sofern die Spiralfeder sich in radialer Richtung verjüngt, statt wie in den Figuren 6a und 6c zu erkennen, ist eine zur Drehachse 4 hin trichterförmige Ausgestaltung anzunehmen, ist der Keilwinkel α andersherum orientiert.

Die Figur 6b zeigt die vergrößerte Teildarstellung des Bereiches B aus der Figur 6a.

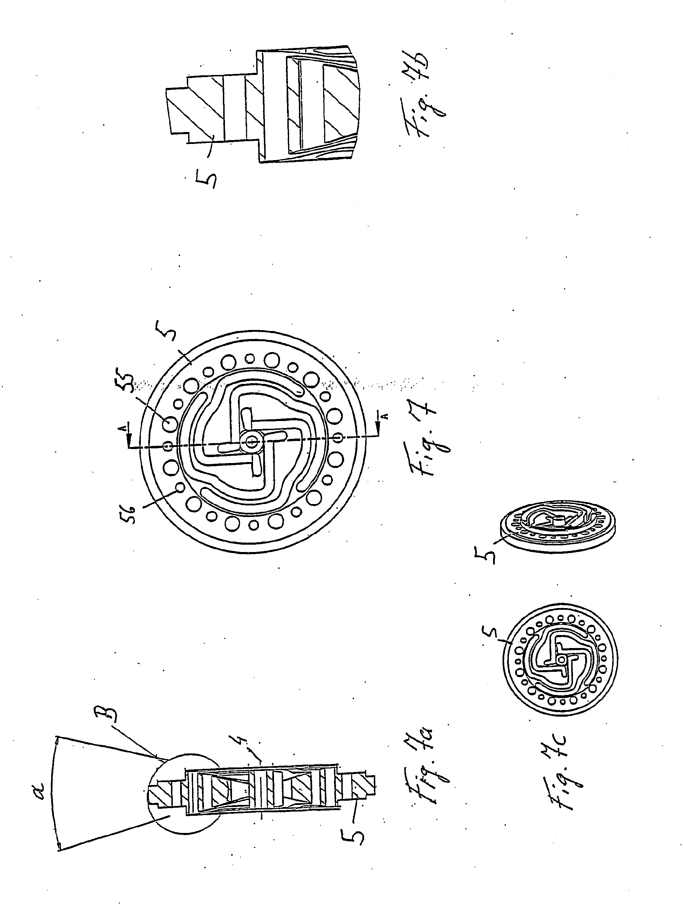

In der Figur 7 ist eine Spiralfeder 5 in Draufsicht mit einer nicht linearen Spiralgeometrie gezeigt. Die Spiralfeder 5 besitzt vier Spiralgänge mit einer Windungslänge von ungefähr 225°, wodurch sich eine wesentlich steifere Federcharakteristik als bei der Spiralfeder 5 der Figur 6 ergibt. Auch bei der Spiralfeder 5 gemäß der Figur 7 sind Bohrungen 55 am Umfang der Spiralfeder 5 verteilt, vorliegend in einer größeren Anzahl als bei der Spiralfeder gemäß der Figur 6. Kleinere Bohrungen 56 dienen zur Materialeinsparung, können aber auch gemeinsam mit Bolzen zur Momentübertragung verwendet werden. In der Figur 7a ist der Keilwinkel α bzw. der Keilwinkel der nicht dargestellten Anlaufflächen 6, 7 zueinander zu erkennen. Die Figur 7c zeigt die napfartige Vertiefung zur Drehachse 4 hin.

Die Figur 8 zeigt in einer Teilschnittdarstellung, ähnlich der der Figur 5, eine alternative Ausgestaltung der Gelenkeinrichtung. Statt einer Übertragung der Reibkräfte ausschließlich an den Kontaktflächen der Spiralfeder 5 an den Anlaufflächen 6, 7 und den außen liegenden Reibflächen 36, 537 sind in dem Unterteil 3 und dem Deckel 53 Keilprofile 30, 530 eingearbeitet, in denen Reiblamellen 531, 532, 533, 31, 32, 33 axial verlagerbar und drehfest eingeführt sind. Anstelle des Keilprofils 30 können auch Bolzen oder andere formschlüssige Verbindungselemente vorgesehen sein. Die drehfeste Lagerung kann durch einen Formschluss von Vorsprüngen auf dem Umfang der Lamellen 31, 32, 33, 531, 532, 533 und Nuten und Kerben in dem Unterteil 3 und Deckel 53 hergestellt sein. Zwischen den Lamellenpaketen 31, 32, 33, 531, 532, 533 sind auf der dem Oberteil 2 zugeordneten Seite der Gelenkeinrichtungen Bolzen 16, 17 zur Momentenübertragung in den Anlaufflächen 6, 7 und dem Bügel 21 und dem Oberteil 2 angeordnet. Auf diesen Bolzen 16, 17 sind Reiblamellen 71, 72, 62, 63 aufgesteckt, wodurch sich eine axial verschiebliche, momentenstarre Verbindung ausbildet. Die Bolzen 16, 17 sind in den Anlaufflächen 6, 7 eingepresst. Wird nun die Spiralfeder 5 in Windungsrichtung verdreht, legen sich die Spiralfedergänge an die Anlauffiächen 6, 7 an und drücken diese nach außen. Dadurch werden die Lamellenpakete aufgrund der axialen Beweglichkeit und der momentenstarren Lagerung in dem Unterteil 3 bzw. an dem nicht dargestellten Oberteil 2 aneinander gepresst und verhindern oder erschweren eine Drehbewegung des Oberteils 2 relativ zu dem Unterteil 3. Eine abschließende Reibpaarung ist durch die Fläche zwischen einem dem Oberteil 2 zugeordneten Abtriebsteil 73, 64 und dem Deckel 53 bzw. dem Unterteil 3 realisiert.

In der dargestellten Ausführungsform werden insgesamt 16 Reibflächenpaarungen zur Verfügung gestellt, wodurch sich die Reibfläche wesentlich erhöhen lässt. Je mehr Lamellen vorgesehen sind, desto größer wird die Reibfläche. Auch bei einer Ausgestaltung der Gelenkeinrichtung mit den Lamellenpakten ist eine Anordnung mehrerer Spiralfedern, gegebenenfalls in entgegengesetzter Wndungsausrichtung möglich. Ebenso sind Anwendungen bei durchdrehenden Komponenten möglich.

## Patentansprüche

1. Gelenkeinrichtung für Orthesen oder Prothesen, mit einem Oberteil (2) und einem um eine Schwenkachse drehbar daran gelagerten Unterteil (3), und einer Bremseinrichtung, die eine Schwenkbewegung des Unterteils relativ zu dem Oberteil abbremst oder blockiert, wobei die Gelenkeinrichtung eine Spiralfeder (5) aufweist, die um eine Verdrehachse (4) parallel zu der Schwenkachse verspannbar gelagert ist, **dadurch gekennzeichnet, dass** die Spiralfeder (5) zwischen zwei Anlaufflächen (6, 7) angeordnet ist und bei Verspannung in Richtung auf die Anlaufflächen (6, 7) mit diesen in Kontakt tritt.

2. Gelenkeinrichtung nach Anspruch 1, **dadurch gekennzeichnet, dass** die Verdrehachse (4) mit der Schwenkachse zusammenfällt.

3. Gelenkeinrichtung nach Anspruch 2, **dadurch gekennzeichnet, dass** die Anlauffläche (6, 7) radial konisch oder radial gekrümmt ausgebildet ist.

4. Gelenkeinrichtung nach Anspruch 3, **dadurch gekennzeichnet, dass** die Spiralfeder (5) korrespondierend zu der Kontur der Anlaufflächen (6, 7) konturiert ist.

5. Gelenkeinrichtung nach einem der Ansprüche 3 oder 4, **dadurch gekennzeichnet, dass** der Konuswinkel (α) der Anlaufflächen (6, 7) zwischen 10° und 50° beträgt.

6. Gelenkeinrichtung nach einem der voranstehenden Ansprüche, **dadurch gekennzeichnet, dass** die Spiralfeder (5) in ihrer Spannung einstellbar ist.

7. Gelenkeinrichtung nach einem der voranstehenden Ansprüche, **dadurch gekennzeichnet, dass** die Spiralfeder (5) in ihrer Ausgangsstellung in Kontakt mit den Anlaufflächen (6, 7) steht.

8. Gelenkeinrichtung nach einem der voranstehenden Ansprüche, **dadurch gekennzeichnet, dass** die Spiralfeder (5) mit einem Aktuator gekoppelt ist, der die Spiralfeder (5) in oder entgegen der Windungsrichtung der Spirale auf die Anlaufflächen (6, 7) verspannt.

9. Gelenkeinrichtung nach Anspruch 8, **dadurch gekennzeichnet, dass** der Aktuator ein Stellmotor ist.

10. Gelenkeinrichtung nach Anspruch 8 oder 9, **dadurch gekennzeichnet, dass** der Aktuator über einen Hebel (40) oder ein Zahnrad oder Zahnradteil mit der Spiralfeder (5) gekoppelt ist.

11. Gelenkeinrichtung nach Anspruch 10, **dadurch gekennzeichnet, dass** der Hebel (40) oder das Zahnrad in Verriegelungsrichtung federbelastet ist.

12. Gelenkeinrichtung nach einem der Ansprüche 8 bis 11, **dadurch gekennzeichnet, dass** die Spiralfeder (5) in ihrer Ausgangsstellung gegen den Aktuator in Verriegelungsrichtung vorgespannt ist.

13. Gelenkeinrichtung nach einem der Ansprüche 8 bis 12, **dadurch gekennzeichnet, dass** eine Sensoreinrichtung (9) vorgesehen ist, die Kräfte, Momente und/oder Winkelstellung der Ober- und Unterteile (2, 3) zueinander misst und mit einer Steuereinheit gekoppelt ist, die in Abhängigkeit von den gemessenen Größen den Aktuator aktiviert und die Spiralfeder (5) verspannt.

14. Gelenkeinrichtung nach einem der Ansprüche 8 bis 13, **dadurch gekennzeichnet, dass** der Aktuator über myoelektrische Signale angesteuert wird.

15. Gelenkeinrichtung nach einem der. Ansprüche 8 bis 14, **dadurch gekennzeichnet, dass** eine Einrichtung zur Erfassung der räumlichen Orientierung des Oberteils und/oder der Unterteils mit dem Aktuator gekoppelt ist.

16. Gelenkeinrichtung nach einem der Ansprüche 8 bis 15, **dadurch gekennzeichnet, dass** eine Einrichtung zur Erfassung der in der Orthese auftretenden Beschleunigungen des Oberteils und/oder des Unterteils oder daran befestigter Einrichtungen mit dem Aktuator gekoppelt ist.

17. Gelenkeinrichtung nach einem der voranstehenden Ansprüche, **dadurch gekennzeichnet, das** zumindest eine Anlauffläche (6, 7) axial verschieblich gelagert ist.

18. Gelenkeinrichtung nach einem der voranstehenden Ansprüche, **dadurch gekennzeichnet, dass** die Spiralfeder (5) mehrgängig ausgebildet ist.

19. Gelenkeinrichtung nach einem der voranstehenden Ansprüche, **dadurch gekennzeichnet, dass** die Spiralfeder (5) eine nichtlineare Spiralgeometrie aufweist.

20. Gelenkeinrichtung nach einem der voranstehenden Ansprüche, **dadurch gekennzeichnet, dass** die Anlaufflächen (6, 7) mit einem Lamellenverband gekoppelt sind, um die Anzahl der Reibflächen zu erhöhen.

## Claims

1. A joint device for orthoses or prostheses, with an upper part (2), with a lower part (3) mounted on the upper part in such a way as to turn about a pivot axis, and with a braking means that brakes or blocks a pivoting movement of the lower part relative to the upper part, the joint device comprises a spiral spring (5) which is mounted such that it can be braced about a rotation axis (4) parallel to the pivot axis, **characterized in that** the spiral spring (5) is arranged between two run-on surfaces (6, 7) and comes into contact with the run-on surfaces (6, 7) when braced in their direction.

2. The joint device as claimed in claim 1, **characterized in that** the rotation axis (4) coincides with the pivot axis.

3. The joint device as claimed in claim 2, **characterized in that** the run-on surface has a radially conical or radially curved design.

4. The joint device as claimed in claim 3, **characterized in that** the spiral spring (5) is contoured in a manner corresponding to the contour of the run-on surfaces (6, 7).

5. The joint device as claimed in one of the claims 3 and 4, **characterized in that** the cone angle (α) of the run-on surfaces (6, 7) is between 10° and 50°.

6. The joint device as claimed in one of the preceding claims, **characterized in that** the bracing of the spiral spring (5) can be adjusted.

7. The joint device as claimed in one of the preceding claims, **characterized in that** the spiral spring (5), in its starting position, is in contact with the run-on surfaces (6, 7).

8. The joint device as claimed in one of the preceding claims, **characterized in that** the spiral spring (5) is coupled to an actuator that braces the spiral spring (5) onto the run-on surfaces (6, 7) in or counter to the direction of winding of the spiral.

9. The joint device as claimed in claim 8, **characterized in that** the actuator is a servomotor.

10. The joint device as claimed in claim 8 or 9, **characterized in that** the actuator is coupled to the spiral spring (5) via a lever (40) or a toothed wheel or part of a toothed wheel.

11. The joint device as claimed in claim 10, **characterized in that** the lever (40) or the toothed wheel is spring-loaded in the direction of locking.

12. The joint device as claimed in one of claims 8 through 11, **characterized in that** the spiral spring (5), in its starting position, is pretensioned counter to the actuator in the direction of locking.

13. The joint device as claimed in one of claims 8 through 12, **characterized in that** a sensor device (9) is provided that measures forces, moments and/or the angle of the upper and lower parts (2, 3) relative to each other and that is coupled to a control unit which, as a function of the measured variables, activates the actuator and braces the spiral spring (5).

14. The joint device as claimed in one of claims 8 through 13, **characterized in that** the actuator is controlled via myoelectric signals.

15. The joint device as claimed in one of claims 8 through 14, **characterized in that** a device for detecting the spatial orientation of the upper part and/or of the lower part is coupled to the actuator.

16. The joint device as claimed in one of claims 8 through 15, **characterized in that** a device for detecting the accelerations of the upper part and/or lower part of the orthosis, or of devices secured thereto, is coupled to the actuator.

17. The joint device as claimed in one of the preceding claims, **characterized in that** at least one run-on surface (6, 7) is mounted so as to be axially displaceable.

18. The joint device as claimed in one of the preceding claims, **characterized in that** the spiral spring (5) is designed with multiple turns.

19. The joint device as claimed in one of the preceding claims, **characterized in that** the spiral spring (5) has a nonlinear spiral geometry.

20. The joint device as claimed in one of the preceding claims, **characterized in that** the run-on surfaces (6, 7) are coupled to an arrangement of plates in order to increase the number of friction surfaces.

## Revendications

1. Dispositif d'articulation pour des orthèses ou des prothèses, comprenant une partie supérieure (2) et une partie inférieure (3) montée rotative sur la partie supérieure autour d'un axe de pivotement, et un dispositif de freinage qui freine ou bloque un mouvement de pivotement de la partie inférieure relativement à la partie supérieure, le dispositif d'articulation présentant un ressort en spirale (5) qui est monté autour d'un axe de déformation rotative (4) en étant serrable parallèlement à l'axe de pivotement, **caractérisé en ce que** le ressort en spirale (5) est agencé entre deux pistes (6, 7) et entre en contact avec celles-ci lors du serrage vers les pistes (6, 7).

2. Dispositif d'articulation selon la revendication 1, **caractérisé en ce que** l'axe de déformation rotative (4) coïncide avec l'axe de pivotement.

3. Dispositif d'articulation selon la revendication 2, **caractérisé en ce que** la piste (6, 7) est formée conique radialement ou courbée radialement.

4. Dispositif d'articulation selon la revendication 3, **caractérisé en ce que** le ressort en spirale (5) est contouré de façon correspondante au contour des pistes (6, 7).

5. Dispositif d'articulation selon l'une des revendications 3 ou 4, **caractérisé en ce que** l'angle de cône α des pistes (6, 7) est compris entre 10° et 50°.

6. Dispositif d'articulation selon l'une des revendications précédentes, **caractérisé en ce que** le ressort en spirale (5) est ajustable en tension.

7. Dispositif d'articulation selon l'une des revendications précédentes, **caractérisé en ce que** le ressort en spirale (5) est en contact avec les pistes (6, 7) dans sa position initiale.

8. Dispositif d'articulation selon l'une des revendications précédentes, **caractérisé en ce que** le ressort en spirale (5) est couplé avec un actuateur qui serre le ressort en spirale (5) contre les pistes (6, 7) dans la direction d'enroulement de la spirale, ou à l'opposé.

9. Dispositif d'articulation selon la revendication 8, **caractérisé en ce que** l'actuateur est un servomoteur.

10. Dispositif d'articulation selon la revendication 8 ou 9, **caractérisé en ce que** l'actuateur est couplé avec le ressort en spirale (5) par l'intermédiaire d'un levier (40) ou une roue dentée ou portion de roue dentée.

11. Dispositif d'articulation selon la revendication 10, **caractérisé en ce que** le levier (40) ou la roue dentée est sollicitée par ressort dans la direction de verrouillage.

12. Dispositif d'articulation selon l'une des revendications 8 à 11, **caractérisé en ce que** dans sa position initiale le ressort en spirale (5) est préserré contre l'actuateur, dans la direction de verrouillage.

13. Dispositif d'articulation selon l'une des revendications 8 à 12, **caractérisé en ce qu'**il est prévu un dispositif capteur (9) qui mesure les forces, moments et/ou positions angulaires des parties inférieure et supérieure (2, 3) l'une par rapport à l'autre, et qui est couplé à une unité de commande qui, en fonction des grandeurs mesurées, active l'actuateur et serre le ressort en spirale (5).

14. Dispositif d'articulation selon l'une des revendications 8 à 13, **caractérisé en ce que** l'actuateur est commandé par l'intermédiaire de signaux myoélectriques.

15. Dispositif d'articulation selon l'une des revendications 8 à 14, **caractérisé en ce qu'**un dispositif pour relever l'orientation spatiale de la partie supérieure et/ou de la partie inférieure, est couplé avec l'actuateur.

16. Dispositif d'articulation selon l'une des revendications 8 à 15, **caractérisé en ce qu'**un dispositif pour relever les accélérations de la partie supérieure et/ou de la partie inférieure et/ou de dispositifs qui y sont fixés, accélérations survenant dans l'orthèse, est couplé avec l'actuateur.

17. Dispositif d'articulation selon l'une des revendications précédentes, **caractérisé en ce qu'**au moins une piste (6, 7) est montée déplaçable axialement.

18. Dispositif d'articulation selon l'une des revendications précédentes, **caractérisé en ce que** le ressort en spirale (5) est formé à pas multiples.

19. Dispositif d'articulation selon l'une des revendications précédentes, **caractérisé en ce que** le ressort en spirale (5) présente une géométrie de spirale non linéaire.

20. Dispositif d'articulation selon l'une des revendications précédentes, **caractérisé en ce que** les pistes (6, 7) sont couplées avec un ensemble de lamelles pour augmenter le nombre des surfaces de frottement.
